(19) Europäisches Patentamt European Patent Office Office européen des brevets

(11) **EP 2 917 884 B1**

(12) **FASCICULE DE BREVET EUROPEEN**

(45) Date de publication et mention de la délivrance du brevet:
**08.05.2019 Bulletin 2019/19**

(21) Numéro de dépôt: **13789792.2**

(22) Date de dépôt: **12.11.2013**

(51) Int Cl.:
*G01W 1/10* [(2006.01)]       *G06F 17/50* [(2006.01)]
*G01N 33/00* [(2006.01)]

(86) Numéro de dépôt international:
**PCT/EP2013/073625**

(87) Numéro de publication internationale:
**WO 2014/072528 (15.05.2014 Gazette 2014/20)**

(54) **PROCEDE DE DETERMINATION DE FLUX DE GAZ DANS UN ESPACE GEOGRAPHIQUE**

VERFAHREN ZUR BESTIMMUNG EINES GASSTROM IN EINEM GEOGRAPHISCHEN GEBIET

METHOD FOR DETERMINING A GAS FLUX IN A GEOGRAPHICAL SPACE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.11.2012 FR 1260732**

(43) Date de publication de la demande:
**16.09.2015 Bulletin 2015/38**

(73) Titulaire: **Commissariat à l'Énergie Atomique et aux Énergies Alternatives 75015 Paris (FR)**

(72) Inventeur: **CHEVALLIER, Frédéric F-75014 Paris (FR)**

(74) Mandataire: **Brevalex 95, rue d'Amsterdam 75378 Paris Cedex 8 (FR)**

(56) Documents cités:
- **W. PETERS ET AL: "An ensemble data assimilation system to estimate CO2 surface fluxes from atmospheric trace gas observations", JOURNAL OF GEOPHYSICAL RESEARCH, vol. 110, D24304, 1 janvier 2005 (2005-01-01), pages 1-18, XP055079774, ISSN: 0148-0227, DOI: 10.1029/2005JD006157**
- **F. CHEVALLIER ET AL: "CO2 surface fluxes at grid point scale estimated from a global 21 year reanalysis of atmospheric measurements", JOURNAL OF GEOPHYSICAL RESEARCH, vol. 115, no. D21, 9 novembre 2010 (2010-11-09), pages 1-17, XP055079797, ISSN: 0148-0227, DOI: 10.1029/2010JD013887**
- **Mike Fisher: "Long Window 4D-Var", Proceedings of the 2011 ECMWF Seminar on Data Assimilation for Atmosphere and Ocean, 7 septembre 2011 (2011-09-07), pages 1-51, XP055079816, Extrait de l'Internet: URL:http://www.ecmwf.int/newsevents/meetings/annual_seminar/2011/presentations/Fisher.pdf [extrait le 2013-09-18]**

**Description**

Le sujet de l'invention présente est un procédé de détermination par localisation ou de cartographie de flux de gaz dans un espace géographique, ces flux de gaz étant des quantités avec lesquelles les gaz considérés apparaissent ou disparaissent dans chaque région de cet espace.

Ces procédés peuvent servir à détecter le lieu d'origine de certains gaz, comme le méthane ou le dioxyde de carbone, produits notamment par les combustions impliquées par les activités humaines. Des flux négatifs, correspondant à une disparition de ces gaz, existent aussi, par exemple grâce à la végétation si elle les absorbe.

La détermination est rendue possible par l'utilisation de modèles numériques qui expriment les concentrations des gaz, dans les régions en lesquelles l'espace géographique est discrétisé, en fonction des flux positifs ou négatifs qui y sont produits, des transports des gaz sous l'action notamment du vent, et des réactions chimiques de combinaisons ou dissociations qui affectent les gaz et équivalent à des flux supplémentaires où les gaz sont encore produits ou détruits sur place ; ces réactions sont toutefois presque inexistantes dans le cas du dioxyde de carbone, qui est un gaz très stable. Les résultats des modèles numériques sont corrélés avec des observations, c'est-à-dire des mesures de la concentration des gaz, à certains endroits de l'espace géographique (ou même hors de cet espace) et à certains moments. Les flux de gaz introduits dans le modèle sont considérés comme optimaux si les concentrations des gaz déduites du modèle correspondent aux observations dans leur intervalle de confiance et si ces flux correspondent à des flux a priori dans leur intervalle de confiance. Autrement dit, les flux sont optimaux s'ils sont le meilleur compromis entre l'accord aux concentrations observées et l'accord aux flux a priori, étant donné la précision de chaque pièce d'information. Mathématiquement, les flux optimaux et leur incertitude sont exprimés par le théorème de Bayes.

Dans la pratique, il existe trois genres de procédés pour déterminer les flux optimaux :

- des procédés analytiques, dans lesquels les modèles sont utilisés directement, en utilisant des formules algébriques ;
- des procédés par ensembles, qui reposent sur des ensembles statistiques de simulations ; et
- des procédés variationnels, qui reposent sur la minimisation d'une fonction dite de coût, qui exprime la somme d'une différence globale entre les observations et les estimations des concentrations et d'une différence globale entre les flux déterminés et les flux a priori.

L'article de W. Peters et al. « An ensemble data assimilation system to estimate CO2 surface fluxes from atmospheric trace gas observations", Journal of Geophysical Research, vol. 110, D24304, 1er janvier 2009, (2009-01-01), p. 1-18, XP 055079774 décrit un procédé par ensembles où les calculs de modélisation sont effectués sur des ensembles de segments temporels, chaque calcul étant mené sur un ensemble dépourvu du segment temporel le plus ancien de l'ensemble précédent mais pourvu d'un nouveau segment temporel situé juste après l'ensemble précédent. Les calculs sont ainsi menés sur des ensembles se recouvrant et successivement décalés vers la fin de la durée d'étude. Chacun des segments temporels est ainsi calculé autant de fois qu'il existe d'ensembles auxquels il appartient, ce qui permet de converger vers une évaluation correcte des concentrations. Les segments temporels n'ont toutefois pas de portion de recouvrement. Les calculs doivent être menés successivement sur chacun des ensembles temporels, et le temps de calcul est donc à peu près proportionnel au nombre d'ensembles de segments temporels, qui peuvent être nombreux en pratique.

Le présent procédé est un perfectionnement de la troisième catégorie (procédés variationnels). Il sera décrit pour un gaz seul, mais il peut être appliqué à plusieurs gaz.

Les procédés variationnels sont ceux qui permettent le mieux d'obtenir des estimations de flux à haute résolution spatio-temporelle et à partir d'un grand nombre de données, l'espace géographique pouvant couvrir la Terre entière et la durée représenter des dizaines d'années, mais les temps de calcul deviennent alors énormes.

Le but principal de l'invention est de diminuer ce temps de calcul et d'obtenir des résultats d'estimation dans des délais acceptables, en permettant d'estimer les flux par des calculs parallèles et simultanés menés indépendamment sur des segments temporels successifs, et de faire une synthèse des calculs dans une étape finale. Une telle segmentation de la durée d'étude est tenue pour impossible dans les procédés variationnels.

Selon l'invention, les modélisations de transport de gaz sont effectuées séparément sur des segments successifs entre lesquels la durée d'étude est divisée, ces segments présentent des portions de recouvrement, et les résultats des modélisations sont exprimés dans chaque segment, sous forme incrémentale de variations de la concentration du gaz étudié.

Les calculs peuvent ainsi être effectués par des processeurs séparés et simultanément sur chacun des segments. Le temps de calcul devient à peu près proportionnel au nombre d'itérations à effectuer, plus réduit que le nombre de segments temporels à considérer. L'assemblage des résultats des segments est facile. Les calculs de corrélation entre les observations et les estimations des concentrations et les itérations sont ensuite accomplis selon les techniques connues. A la différence de la modélisation du transport de gaz, ces calculs de corrélation sont effectués à un niveau supérieur du procédé, sans que la période d'étude soit segmentée, une fois les segments assemblés d'après les modalités expliquées plus loin. La cohérence physique et statistique du modèle est bien respectée du début à la fin de la période d'étude pour l'assemblage correct des segments.

Selon certaines caractéristiques optionnelles mais souvent avantageuses, les portions de recouvrement sont d'autant plus longues que l'espace géographique est vaste et le transport du gaz dans l'espace est lent; les résultats des modélisations de transport du gaz sont exploités pour estimer les concentrations du gaz en excluant une portion de recouvrement au début de chacun des segments, pour chacun des segments sauf un premier des segments, dont les résultats sont complètement exploités; et le procédé comporte une addition d'un terme spatialement uniforme aux résultats de chacun des segments sauf le premier des segments, de manière à faire coïncider les résultats desdits segments avec les résultats des segments précédents obtenus simultanément aux portions de recouvrement.

Une réalisation purement illustrative des différents détails et aspects de l'invention sera maintenant décrite complètement au moyen des figures suivantes :

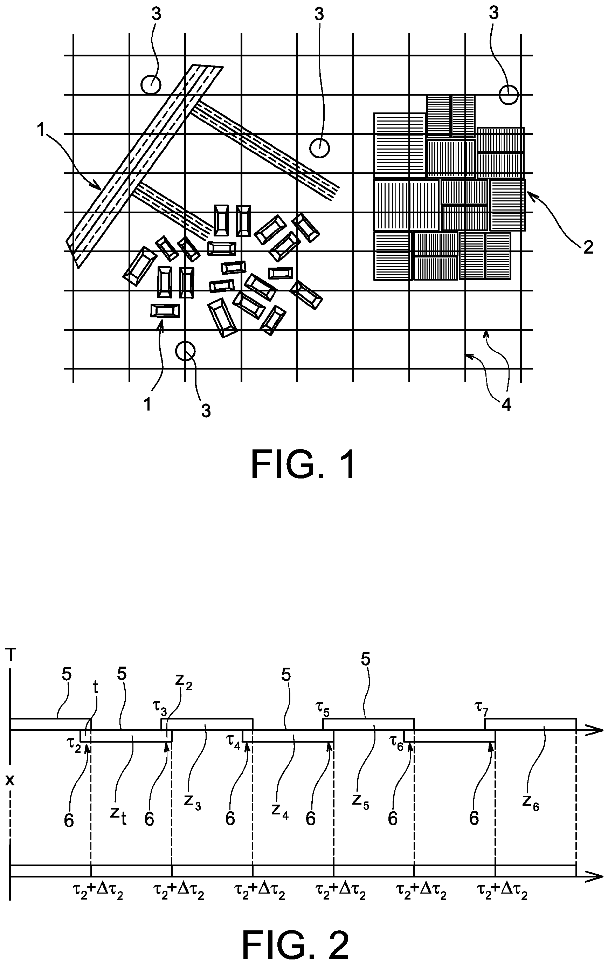

- la modélisation de la Figure 1 exprime les facteurs du problème à résoudre et la modélisation d'un espace géographique ;

- et la Figure 2 explicite les aspects fondamentaux de l'invention.

Se référant à la Figure 1, on voit un espace géographique où des flux d'un gaz déterminé peuvent apparaître et qui comprend notamment des lieux 1 où ces flux apparaissent de façon privilégiée, éventuellement des lieux 2 où ils sont absorbés, et des stations 3 d'observation, qui mesurent les concentrations du gaz. Les stations 3 peuvent être placées dans l'espace géométrique même où les flux apparaissent ou sont absorbés, et aussi près de cet espace. Un maillage 4 couvre tout l'espace géographique considéré et le divise en parcelles pour les besoins de la modélisation.

Dans la suite de cette description, on appelle $\mathbf{x}$ les variables à déterminer (les flux de gaz, exprimés en un vecteur), $\mathbf{x}^a$ la valeur optimale de ces variables $\mathbf{x}$ et $\mathbf{A}$ la covariance de la matrice des erreurs de $\mathbf{x}$, les variables $\mathbf{x}$ étant exprimées sous forme bayésienne d'une distribution de probabilités, $\mathbf{x}^b$ une estimation a priori des variables et $\mathbf{B}$ sa matrice de covariance, $H$ le modèle numérique d'évolution des flux, $\mathbf{H}$ sa matrice jacobienne (dont les coefficients correspondent aux valeurs locales des dérivées partielles du modèle $H$) et $\mathbf{R}$ sa matrice d'erreurs. Des paramètres du modèle comprennent des mesures ou d'autres estimations de phénomènes météorologiques, tels que vitesse et direction des vents, température, pression, etc. pour évaluer le transport du gaz considéré dans l'espace géographique et à ses limites. La théorie de l'estimation optimale indique que $\mathbf{x}^a$ correspond au minimum de la fonction de coût $J(\mathbf{x})$, où

$$J(\mathbf{x}) = \frac{1}{2}(\mathbf{x} - \mathbf{x}^b)^{\mathrm{T}}\mathbf{B}^{-1}(\mathbf{x} - \mathbf{x}^b) + \frac{1}{2}(H(\mathbf{x}) - \mathbf{y})^{\mathrm{T}}\mathbf{R}^{-1}(H(\mathbf{x}) - \mathbf{y}) \qquad (1)$$

La recherche de ce minimum implique des itérations où l'inconnue $\mathbf{x}$ est modifiée à chaque fois, et qui implique des calculs de gradient de la fonction de coût $J$, soit

$$\nabla J(\mathbf{x}) = \mathbf{B}^{-1}(\mathbf{x} - \mathbf{x}^b) + \mathbf{H}^{\mathrm{T}}\mathbf{R}^{-1}(H(\mathbf{x}) - \mathbf{y}) \qquad (2)$$

Enfin, la matrice A peut être calculée d'après

$$\mathbf{A} = (\nabla^2 J(\mathbf{x}))^{-1} \qquad (3)$$

L'inventeur a démontré (F. CHEVALLIER et autres : « Inferring CO2 sources and sinks from satellite observations : Method and application to TOVS data », dans Journal of Geographical Research, vol. 110, D 24309, 29 décembre 2005) l'application de ces principes.

Le procédé présent exploite une linéarisation de l'opérateur numérique $H$. Il est en effet possible de démontrer que

$$\delta\mathbf{c}(t) = \sum_{t'=T}^{t} \mathbf{H}_{t't}^{\varphi} \cdot \delta\boldsymbol{\varphi}(t') + \mathbf{H}_{t't}^{C} \cdot \delta\mathbf{c}(T) \qquad (4)$$

où $\delta\mathbf{c}(t)$ est le vecteur qui contient les incréments de concentration à l'instant t, $\delta\varphi(t')$ le vecteur qui contient les incréments de flux et les incréments des conditions aux limites latérales (apparitions et disparitions du gaz dans l'espace géographique, et entrées et sorties du gaz aux limites de l'espace géographique où le calcul est fait) ; $\mathbf{H}_{t't}^{\varphi}$ est l'opérateur

linéaire qui relie les incréments de concentration à l'instant considéré, aux incréments de flux et aux incréments de conditions aux limites latérales; $\mathbf{H}_{t't}^{C}$ est l'opérateur linéaire qui relie les incréments de concentration à l'instant considéré, aux incréments de concentration à l'origine. $\mathbf{H}_{t't}^{\varphi}$ et $\mathbf{H}_{t't}^{C}$ sont des blocs de la matrice générale **H.**

L'invention comprend la division du calcul en segments 5 successifs, représentés à la Figure 2, qui est un diagramme d'axe des temps. Chacun des segments 5 possède une origine $\tau$, de sorte qu'à l'intérieur de chacun d'eux, l'expression précédente devient

$$\delta\mathbf{c}(t) = \sum_{t'=\tau}^{t} \mathbf{H}_{t't}^{\varphi} \cdot \delta\boldsymbol{\varphi}(t') + \mathbf{H}_{t't}^{C} \cdot \delta b(\tau, t) \qquad (5)$$

où $\delta b(\tau, t)$ est un scalaire représentant le destin moyen, à l'instant t, de l'incrément de concentration qui existait au temps $\tau$. Il dépend du temps t à cause de la possibilité de combinaison ou de destruction chimique du gaz. Si toutefois le gaz est inactif chimique, comme c'est le cas du $CO_2$, $\delta b(\tau, t)$ ne dépend pas de t et reste donc constant sur tout le segment.

La cohérence physique et statistique du procédé global dépend d'un traitement correct de ce terme $\mathbf{H}_{t't}^{C} \cdot \delta b(\tau, t)$.

Le modèle numérique **H** étant connu, il est facile de calculer les valeurs $\delta\mathbf{c}(t)$ à l'intérieur de chacun des segments 5, les $\delta\varphi(t')$ étant connus, puis d'assembler les segments pour déterminer l'évolution complète de $\delta\mathbf{c}(t)$ sur toute la durée d'étude, d'obtenir les estimations des concentrations **c** du gaz sur toutes les régions du modèle et à tous les instants et de mener les calculs précédents sur la fonction de coût. L'assemblage des segments revient alors à évaluer les variations de concentration $\delta\mathbf{c}(t)$ sur toute la durée de l'étude, puis à estimer les concentrations **c**(t) d'après des sommes de ces variations. Le modèle numérique est appliqué à nouveau sur les segments par itérations successives en faisant converger les estimations de flux vers les valeurs minimisant la fonction de coût. En effet, les calculs de la formule (5) peuvent être menés indépendamment les uns des autres sur chacun des segments 5. Toutefois, les segments 5 possèdent des périodes de recouvrement 6 de durée $\Delta\tau$, dont la raison s'explique par l'existence du deuxième terme, à savoir le coefficient $\delta b(\tau, t)$.

Deux cas peuvent se présenter. Dans un espace géographique sans condition aux limites latérales d'entrée ou de sortie du gaz, ce qui est le cas notamment d'un modèle couvrant la Terre entière, le terme $\delta b(\tau, t)$ de chaque segment 5 après le premier est obtenu du segment 5 précédent en moyennant des valeurs de $\delta\mathbf{c}(\tau)$. Par exemple, pour le deuxième segment commençant à l'instant $\tau2$, les valeurs de $\delta\mathbf{c}(\tau)$ obtenues dans le premier segment commençant à l'instant $\tau2$ sont moyennées pour toutes les régions considérées de l'espace géographique, pour obtenir $\delta b(\tau_2, \tau_2)$ qui est ensuite exploité dans tout le reste du deuxième segment comme terme correctif. $\delta b(\tau, t)$ peut être constant dans le temps ou corrigé de l'absorption chimique comme dans le cas du méthane. On procédera de même à tous les recouvrements 6 suivants. Les recouvrements 6 des segments 5 assurent donc une liaison entre les calculs des $\delta\mathbf{c}(t)$ sur toute la durée d'étude. Le choix d'une valeur $\delta b(\tau, t)$ constante sur l'espace géographique traduit l'hypothèse que les déplacements du gaz à partir d'un point quelconque peuvent l'amener à n'importe quel autre point du modèle après un temps suffisant, et qu'une répartition stable est plausible et susceptible de conduire à des résultats statistiquement satisfaisants.

Pour les modèles comprenant des conditions aux limites latérales, c'est-à-dire des interactions comprenant des mouvements du gaz entre l'espace géographique considéré et les espaces voisins, la situation est encore plus simple, puisque le terme $\delta b(\tau, t)$ peut complètement être négligé. On peut en effet considérer que le renouvellement de l'atmosphère dans chacune des régions de l'espace géographique est complet après un temps suffisant, de sorte que l'influence de l'origine ($\tau$) est devenue inexistante. Les calculs entre les différents segments 5 sont alors supposés complètement indépendants, et leurs valeurs ne sont retouchées par aucun terme correctif; les recouvrements 6 restent malgré tout nécessaires, puisque les débuts des segments 5 restent soumis aux conditions d'origine, qui servent à faire démarrer les calculs, mais les résultats obtenus au début des segments 5 pendant ces recouvrements 6 ne sont pas pris en considération.

La durée des périodes de recouvrement 6 est choisie en considérant qu'un recouvrement trop bref peut conduire à des erreurs de calculs notables et qu'un recouvrement trop long mène à des calculs plus importants sans nécessité.

Dans un mode de réalisation particulier, on a utilisé le procédé variationnel du présent Inventeur, indiqué dans l'Article mentionné plus haut. Le modèle de transport utilisé était le modèle général de cirulation LBDZ connu dans l'art. Les flux étaient estimés sur une grille de modélisation mondiale (maillage régulier de 3,75° par 2,5°), à une résolution temporelle de huit jours, en séparant le jour et la nuit. Les flux a priori comprenaient des estimations des émissions anthropiques annuelles, des flux océaniques climatologiques, des émissions dues à la combustion de la biomasse, et des flux échangés entre la biosphère et l'atmosphère. Les observations étaient des fractions molaires du $CO_2$ dans l'air sec, collectées par flacon sur terre et en mer, et enregistrées dans les archives du NOAA Earth System Research Laboratory, pour la période entre 1979 et 2010. La durée des segments était de quinze mois, depuis octobre de chaque année jusqu'à décembre de l'année suivante avec trois mois de recouvrement, à l'exception du premier segment, qui durait douze mois, pendant toute la première année d'études (1979).

Les calculs ont pu être menés en quelques jours grâce à l'invention, alors qu'il aurait fallu plusieurs mois sans cette mise en parallèle des calculs sur les segments 5. Un procédé traditionnel a toutefois été mis en oeuvre, sur une durée réduite de 1979 à 1992. Les différences entre les résultats qu'il donnait et ceux de l'invention étaient réduites (20 % au plus). Un second calcul, effectué d'après le procédé de l'invention, mais avec des segments de dix-huit mois, dont six mois de recouvrement, donna des résultats très peu différents du précédent, de sorte qu'on peut estimer qu'un recouvrement de trois mois entre segments 5 temporels, était suffisant ici.

Dans le cas d'un espace géographique par exemple de la taille d'une ville, le recouvrement sera au plus de quelques jours. Le recouvrement sera plus court si les phénomènes météorologiques (en particulier le transport des gaz) sont rapides dans l'espace considéré. A l'inverse, le recouvrement sera plus long si les phénomènes météorologiques sont lents.

Ces valeurs de recouvrement sont valables, même pour des segments de plusieurs mois.

Le procédé de l'invention est en principe utilisable pour tout modèle utilisé dans l'art. Les modèles connus se distinguent les uns des autres notamment par les phénomènes météorologiques et physiques pris en compte (vent, température, pression, convection, échanges avec l'extérieur et notamment la haute atmosphère), la discrétisation de l'espace (horizontalement et verticalement, le modèle pouvant être appliqué à plusieurs couches successives au-dessus de la surface du sol), et l'opérateur numérique choisi (eulérien ou lagrangien par exemple). Ils s'efforcent de décrire les transports du gaz, avec une précision qui dépend notamment du nombre de paramètres qu'ils utilisent, du volume d'observations disponible et d'hypothèses faites pour suppléer aux absences de données mesurées ou connues. La fonction de coût peut être exprimée de plusieurs façons ; elle est d'autant plus élevée que les différences entre les estimations des concentrations et les mesures (observations) de ces mêmes concentrations le sont aussi.

**Revendications**

1. Procédé de détermination de flux de gaz, apparaissant ou disparaissant dans un espace géographique, comprenant :

   - une estimation a priori des flux ;
   - des mesures (8) de concentrations (c) du gaz ;
   - des mesures de phénomènes météorologiques, comprenant des directions et des vitesses de vents dans l'espace géographique, dans une durée d'étude entre des temps de détermination des flux et des temps des mesures ;
   - des applications d'un modèle numérique (H) donnant des estimations des concentrations du gaz aux temps des mesures ($y_1$, $y_2$, ...) d'après des estimations des flux et des modélisations de transport du gaz dans l'espace géographique selon les phénomènes météorologiques mesurés ; lesdites applications étant itératives pour faire converger les estimations des flux vers des flux réels ;

   les applications du modèle numérique étant effectuées séparément sur des segments (5) successifs entre lesquels la durée d'étude est divisée, les segments présentant des portions de recouvrement (6), **caractérisé en ce que** les applications du modèle numérique sont effectuées simultanément sur chacun des segments et ont des résultats exprimés en variations de concentration (ôc(t)) du gaz ; et une estimation de concentration du gaz est calculée, et corrélée aux mesures (8), pour chaque itération des applications, en déterminant une évolution complète des variations de concentration sur toute la durée d'étude.

2. Procédé de détermination de flux de gaz suivant la revendication 1, **caractérisé en ce que** les portions de recouvrement sont d'autant plus longues que l'espace géographique est vaste et le transport du gaz dans l'espace est lent.

3. Procédé de détermination de flux de gaz suivant la revendication 1 ou 2, **caractérisé en ce que** les résultats des modélisations de transport du gaz sont exploités pour estimer les concentrations du gaz en excluant une portion de recouvrement au début de chacun des segments, pour chacun des segments sauf un premier des segments, dont les résultats sont complètement exploités.

4. Procédé de détermination de flux de gaz suivant la revendication 3, **caractérisé en ce qu'**il comporte une addition d'un terme spatialement uniforme (δb(t)) aux résultats de chacun des segments sauf le premier des segments, de manière à faire coïncider les résultats desdits segments avec les résultats des segments précédents obtenus simultanément aux portions de recouvrement (6).

**5.** Procédé de détermination de flux de gaz suivant l'une quelconque des revendications précédentes, **caractérisé en ce que** les variations de concentration du gaz sont estimées par

$$\delta \mathbf{c}(t) = \sum_{t'=\tau}^{t} \mathbf{H}_{t't}^{\varphi} \cdot \delta \boldsymbol{\varphi}(t') + \mathbf{H}_{t't}^{C} \cdot \delta b(\tau, t)$$

où t est le temps, $\tau$ un temps d'origine d'un segment, $\mathbf{H}_{t't}^{\varphi}$ et $\mathbf{H}_{t't}^{C}$ les opérateurs linéarisés du modèle (*H*) à un temps et un lieu déterminés, $\delta\varphi$ des incréments de flux et de conditions aux limites, $\delta\mathbf{c}$ les variations de concentration, et $\delta b$ est un scalaire.

**6.** Procédé de détermination de flux de gaz suivant la revendication 5, **caractérisé en ce que**, pour un premier des segments, les variations de concentration du gaz sont estimées par

$$\delta \mathbf{c}(t) = \sum_{t'=T}^{t} \mathbf{H}_{t't}^{\varphi} \cdot \delta \boldsymbol{\varphi}(t') + \mathbf{H}_{t't}^{C} \cdot \delta \mathbf{c}(T)$$

où T est une origine de la durée d'étude.

**Patentansprüche**

**1.** Verfahren zum Ermitteln von Gasflüssen, die in einem geografischen Raum auftreten oder versiegen, umfassend:

- apriorisches Schätzen der Flüsse;
- Messungen (8) der Gaskonzentrationen (c);
- Messungen meteorologischer Erscheinungen, einschließlich Windrichtungen und -geschwindigkeiten im geografischen Raum, für einen Untersuchungszeitraum zwischen Flussermittlungszeiten und Messzeiten;
- Anwendungen eines numerischen Modells *(H)*, das Schätzungen der Gaskonzentrationen zu den Messzeiten (y1, y2, ...) widerspiegelt, basierend auf Schätzungen und Modellierungen des Gastransports im geografischen Raum gemäß gemessenen meteorologischen Erscheinungen; wobei die Anwendungen iterativ sind, um Schätzungen der Flüsse mit realen Flüssen zusammenzuführen;

wobei die Anwendungen des numerischen Modells getrennt für aufeinanderfolgende Segmente (5) durchgeführt werden, zwischen denen der Untersuchungszeitraum aufgeteilt ist, wobei die Segmente überlappende Bereiche (6) aufweisen,
**dadurch gekennzeichnet, dass**
die Anwendungen des numerischen Modells gleichzeitig an jedem der Segmente durchgeführt werden und Ergebnisse haben, die in Gaskonzentrationsschwankungen ($\delta c(t)$) ausgedrückt werden; wobei für jede Iteration der Anwendungen eine Gaskonzentrationsschätzung berechnet und mit den Messungen (8) korreliert wird, indem ein vollständiger Verlauf der Konzentrationsschwankungen über den gesamten Untersuchungszeitraum ermittelt wird.

**2.** Verfahren zum Ermitteln von Gasflüssen nach Anspruch 1,
**dadurch gekennzeichnet, dass**
die Überlappungsbereiche umso länger sind, als der geographische Raum groß ist und der Gastransport im Raum langsam ist.

**3.** Verfahren zum Ermitteln von Gasflüssen nach Anspruch 1 oder 2,
**dadurch gekennzeichnet, dass**
die Ergebnisse der Gastransportmodellierungen zum Schätzen der Gaskonzentrationen ausgewertet werden, indem ein Überlappungsbereich am Anfang eines jeden der Segmente für jedes der Segmente mit Ausnahme eines ersten der Segmente ausgeschlossen wird, dessen Ergebnisse vollständig ausgewertet werden.

**4.** Verfahren zum Ermitteln von Gasflüssen nach Anspruch 3,
**dadurch gekennzeichnet, dass**
es das Hinzufügen eines räumlich gleichförmigen Terms ($\delta b(t)$) zu den Ergebnissen jedes der Segmente mit Ausnahme des ersten der Segmente umfasst, so dass die Ergebnisse der Segmente mit den Ergebnissen der vorherigen Segmente in Übereinstimmung gebracht werden, die gleichzeitig an den Überlappungsbereichen (6) erhalten werden.

5. Verfahren zum Ermitteln von Gasflüssen nach einem der vorangehenden Ansprüche,
**dadurch gekennzeichnet, dass**
die Gaskonzentrationsschwankungen geschätzt werden durch

$$\delta\mathbf{c}(t) = \sum_{t'=\tau}^{t} \mathbf{H}_{t't}^{\varphi} \cdot \delta\boldsymbol{\varphi}(t') + \mathbf{H}_{t't}^{C} \cdot \delta b(\tau, t)$$

worin t die Zeit ist, $\tau$ ein Anfangszeitpunkt eines Segments ist, $H_{t't}^{\varphi}$ und $H_{t't}^{C}$ die linearisierten Operatoren des Modells (H) zu einem bestimmten Zeitpunkt und an einem bestimmten Ort sind und $\delta_{\varphi}$ Inkremente der Flüsse und Randbedingungen sind, $\delta c$ Konzentrationsschwankungen sind und $\delta b$ ein Skalar ist.

6. Verfahren zum Ermitteln von Gasflüssen nach Anspruch 5,
**dadurch gekennzeichnet, dass**
für ein erstes der Segmente die Gaskonzentrationsschwankungen geschätzt werden durch

$$\delta\mathbf{c}(t) = \sum_{t'=T}^{t} \mathbf{H}_{t't}^{\varphi} \cdot \delta\boldsymbol{\varphi}(t') + \mathbf{H}_{t't}^{C} \cdot \delta\mathbf{c}(T)$$

worin T der Anfangspunkt des Untersuchungszeitraums ist.

**Claims**

1. A method for determining gas flows, appearing or disappearing in a geographical space, comprising:

   - a priori estimating the flows;
   - measuring (8) gas concentrations (**c**);
   - measuring meteorological phenomena, comprising wind directions and velocities in the geographical space, within a study duration between flow determining times and measuring times;
   - applying a numerical model (*H*) giving estimations of gas concentrations at the measuring times ($y_1$, $y_2$, ...) based on flow estimations and modelizations of gas transportation in the geographical space according to the measured meteorological phenomena; said applications being iterative in order to converge the flow estimations towards real flows;

   the modelizations of gas transportation being separately carried out on successive segments (5) between which the study duration is divided, the segments having overlay portions (6),
   **characterized in that** the applications of the numerical model are made simultaneously on each of the segments and have results expressed as variations in gas concentration ($\delta\mathbf{c}(t)$) ; and an estimate of gas concentration is computed, and correlated to the measurements (8), for each iteration of the applications, by determining a complete evolution of the concentration variations for the entire study duration.

2. The method for determining gas flows according to claim 1, **characterized in that** the overlay portions are all the longer that the geographical space is huge and the gas transportation in space is slow.

3. The method for determining gas flows according to claim 1 or 2, **characterized in that** the modelization results of gas transportation are exploited in order to estimate the gas concentrations excluding an overlay portion at the beginning of each one of the segments, for each one of the segments except for a first one of the segment, the results of which are fully exploited.

4. The method for determining gas flows according to claim 3, **characterized in that** it comprises adding a spatially uniform term ($\delta b(t)$) to the results of each one of the segments except the first one of the segments, so as to match the results of said segments with the results of the previous segments obtained simultaneously with the overlay portions (6).

5. The method for determining gas flows according to any of the preceding claims, **characterized in that** the variations in gas concentration are estimated by

$$\delta \mathbf{c}(t) = \sum_{t'=\tau}^{t} \mathbf{H}_{t't}^{\varphi} \cdot \delta \boldsymbol{\varphi}(t') + \mathbf{H}_{t't}^{C} \cdot \delta b(\tau, t)$$

where t is the time, $\tau$ is an origin time of a segment, $\mathbf{H}_{t't}^{\varphi}$ and $\mathbf{H}_{t't}^{C}$ the linearized operators of the model (H) at a determined time and place, $\delta \varphi$ increments of flows and boundary conditions, $\delta \mathbf{c}$ the variations in concentration, and $\delta b$ is a scalar.

6. The method for determining gas flows according to claim 5, **characterized in that**, for a first one of the segments, the variations in gas concentration are estimated by

$$\delta \mathbf{c}(t) = \sum_{t'=T}^{t} \mathbf{H}_{t't}^{\varphi} \cdot \delta \boldsymbol{\varphi}(t') + \mathbf{H}_{t't}^{C} \cdot \delta \mathbf{c}(T)$$

where T is an origin of the study duration.

# FIG. 1

# FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **W. PETERS et al.** An ensemble data assimilation system to estimate CO2 surface fluxes from atmospheric trace gas observations. *Journal of Geophysical Research,* 01 Janvier 2009, vol. 110 (D24304), 1-18

- **F. CHEVALLIER.** Inferring CO2 sources and sinks from satellite observations : Method and application to TOVS data. *Journal of Geographical Research,* 29 Décembre 2005, vol. 110, 24309